# EUROPEAN PATENT SPECIFICATION

(11) **EP 2 160 190 B1**
(45) Date of publication and mention of the grant of the patent: **04.11.2015**
(21) Application number: 08760508.5
(22) Date of filing: 04.06.2008
(51) Int. Cl.: A61K 31/58, A61P 9/14, A61P 17/02, A61P 1/16

(54) **5BETA, 14BETA-ANDROSTANE DERIVATIVES USEFUL FOR THE TREATMENT OF DISEASES DUE TO ORGAN FIBROSIS**
5BETA; 14BETA-ANDROSTAN-DERIVATE ZUR BEHANDLUNG VON ERKRANKUNGEN DURCH ORGANFIBROSE
DÉRIVÉS DE 5BETA, 14BETA-ANDROSTANE UTILES POUR TRAITER DES MALADIES CAUSÉES PAR UNE FIBROSE DES ORGANES.

(30) Priority: 07.06.2007 EP 07109779
(43) Date of publication of application: 10.03.2010
(73) Proprietor: SIGMA-TAU Industrie Farmaceutiche Riunite S.p.A., 00144 Rome (IT)
(72) Inventor: BERRINO, Liberato, I-80053 Castellamare Di Stabia (IT); CASCINO, Antonio, I-80121 Napoli (IT); CIPOLLARO, Marilena, I-80121 Napoli (IT); FORTE, Amalia, I-80132 Napoli (IT); ROSSI, Francesco, I-80138 Napoli (IT); BIANCHI, Giuseppe, I-20133 Milano (IT); FERRARI, Patrizia, I-21100 Varese (VA) (IT)
(74) Representative: Spadaro, Marco
(86) International application number: PCT/EP2008/056928
(87) International publication number: WO 2008/148812

(56) References cited:
- EP-A- 0 583 578
- EP-A- 0 590 272
- WO-A-2004/089416
- WO-A-2006/044916
- FORTE A ET AL: "PST 2238 treatment affects gene expression in arteriotomy-injured Milan hypertensive rat strain carotids" JOURNAL OF HYPERTENSION, vol. 23, no. Suppl. 2, June 2005 (2005-06), page S244, XP008085925 & 15TH EUROPEAN MEETING ON HYPERTENSION; MILAN, ITALY; JUNE 17 -21, 2005 ISSN: 0263-6352
- QUADRI L ET AL: "17[beta]-(3-Furyl)-5[beta]-androstane-3[b eta]-14[beta],17[alpha]-triol (PST 2238). A very potent antihypertensive agent with a novel mechanism of action" JOURNAL OF MEDICINAL CHEMISTRY, AMERICAN CHEMICAL SOCIETY. WASHINGTON, US, vol. 40, no. 11, 1997, pages 1561-1564, XP002377899 ISSN: 0022-2623

## Description

### FIELD OF THE INVENTION

The present invention relates to 17beta-(3-furyl) and (4-pyridazinyl)-5beta, 14beta-androstane derivatives, as useful agents for preparing a medicament for the prevention and treatment of diseases due to organ fibrosis.

More specifically the invention relates to compounds of formula (I): 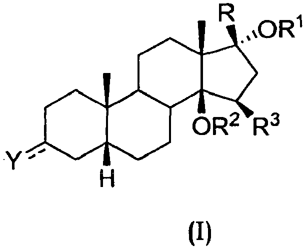 wherein:
the symbol - - - represents a single or a double bond;
Y is oxygen or guanidinoimino when - - - in position 3 is a double bond;
Y is hydroxy, OR⁴ or SR⁴, when - - - in position 3 is a single bond and can have an alpha or beta configuration;
R is an unsubstituted or substituted 3-furyl or 4-pyridazinyl group;
R¹ is hydrogen; methyl; ethyl or n-propyl substituted by OH or NR⁵R⁶;
R² is hydrogen or together to R³ is a bond of an oxirane ring;
R³ is hydrogen or together to R² is a bond of an oxirane ring;
R⁴ is hydrogen; methyl; C2-C6 alkyl or C3-C6 alkenyl or C2-C6 acyl, these alkyl, alkenyl and acyl groups being unsubstituted or substituted by a quaternary ammonium group or one or more OR⁷, NR⁸R⁹, formyl, amidino, guanidinoimino or by NR⁸R⁹ and hydroxy;
R⁵, R⁶ are independently hydrogen; methyl; C2-C6 alkyl unsubstituted or substituted by one NR¹⁰R¹¹, or NR¹⁰R¹¹ and hydroxy, or R⁵ and R⁶ taken together with the nitrogen atom form an unsubstituted or substituted saturated or unsaturated penta- or hexa-monoheterocyclic ring, optionally containing another heteroatom chosen from oxygen or sulfur or nitrogen;
R⁷ is hydrogen, methyl or C2-C4 alkyl, this alkyl being unsubstituted or substituted by one or more NR¹⁰R¹¹ or by NR¹⁰R¹¹ and hydroxy;
R⁸, R⁹ are independently hydrogen; methyl; C2-C6 alkyl or C3-C6 alkenyl, these alkyl and alkenyl groups being unsubstituted or substituted by one or more NR¹⁰R¹¹, or NR¹⁰R¹¹ and hydroxy, or R⁸ and R⁹ taken together with the nitrogen atom form an unsubstituted or substituted saturated or unsaturated penta- or hexa-monoheterocyclic ring, optionally containing another heteroatom chosen from oxygen or sulfur or nitrogen, or R⁸ is hydrogen and R9 is amidino; or NR⁸R⁹ represents propargylamino,
R¹⁰, R¹¹ are independently hydrogen, C1-C6 alkyl, or R¹⁰ and R¹¹, taken together with the nitrogen atom form a saturated or unsaturated penta- or hexa-monoheterocyclic ring.

Also included in this invention are pharmaceutically acceptable salts of (I), which retain the biological activity of the base and are derived from such known pharmaceutically acceptable acids such as hydrochloric, sulfuric, phosphoric, malic, tartaric, maleic, citric, methanesulfonic or benzoic acid.

The alkyl and alkenyl groups may be branched or straight chain groups.

The C1-C6 alkyl group is preferably a C1-C4 alkyl group, e.g. methyl, ethyl, n-propyl, isopropyl, n-butyl, sec-butyl.

The C2-C6 alkyl group is preferably a C2-C4 alkyl group, e.g. ethyl, n-propyl, isopropyl, n-butyl, sec-butyl.

The C3-C6 alkenyl group is preferably a C3-C4 alkenyl group, e.g. 2-propenyl, 2-butenyl.

The C2-C6 acyl is preferably a C2-C4 acyl group, e.g. acetyl, propionyl, butyryl.

The quaternary ammonium group is preferably a trimethylammonium- or a N-methylpyrrolidinium- or a N-methylpiperidinium- group.

The OR⁷ group is preferably hydroxy, 2-aminoethoxy, 3-aminopropoxy, 2-dimethylaminoethoxy, 2-diethylaminoethoxy, 3-dimethylaminopropoxy, 3-amino-2-hydroxypropoxy, 2,3-diaminopropoxy, 2-(1-pyrrolidinyl)ethoxy, 3-(1-pyrrolidinyl)propoxy.

The NR⁵R⁶ group is preferably amino, methylamino, ethylamino, n-propylamino, dimethylamino, diethylamino, pyrrolidinyl, morpholino, piperazinyl, 1-imidazolyl, 2-aminoethylamino, 3-aminopropylamino.

The NR⁸R⁹ group is preferably amino, methylamino, ethylamino, n-propylamino, iso-propylamino, allylamino, propargylamino, dimethylamino, diethylamino, pyrrolidinyl, morpholino, piperazinyl, 1-imidazolyl, 1-guanidino, 2-aminoethylamino, 3-aminopropylamino, 2-(1-pyrrolidinyl)ethylamino, 3-(1-pyrrolidinyl)propylamino, 3-amino-2-hydroxypropylamino, 3-(1-pyrrolidinyl)2-hydroxypropylamino, 2,3-diaminopropylamino, (2-(1-pyrrolidinyl)ethyl)methylamino.

Preferred examples of specific compounds according to the present invention are:
17 beta -(3-Furyl)-5 beta -androstane-3 beta, 14 beta, 17 alpha -triol;
3 beta -(2-Hydroxyethoxy)-17 beta -(3-furyl)-5 beta-androstane-14 beta, 17 alpha -diol;
3 beta -(2-Aminoethoxy)-17 beta -(3-furyl)-5 beta -androstane-14 beta, 17 alpha -diol;
3 beta -(3-Aminopropoxy)-17 beta -(3-furyl)-5 beta-androstane-14 beta, 17 alpha -diol;
3 beta -(2-Methylaminoethoxy)-17 beta -(3-furyl)-5 beta-androstane-14 beta ,17 alpha -diol;
3 beta -(2-(1-Pyrrolidinyl)ethoxy)-17 beta -(3-furyl)-5 beta-androstane-14 beta, 17 alpha -diol;
3 beta -(2-(3-(1-Pyrrolidinyl)propoxy)ethoxy)-17 beta -(3-furyl)-5 beta -androstane-14 beta, 17 alpha -diol;
3 beta -(3-(1-Pyrrolidinyl)propoxy)-17 beta -(3-furyl)-5 beta-androstane-14 beta, 17 alpha-diol;
3 beta -(2-(1-Imidazolyl)ethoxy)-17 beta -(3-furyl)-5 beta-androstane-14 beta, 17 alpha -diol;
3 beta -(2-(2-Imidazolin-2-yl)ethoxy)-17 beta -(3-furyl)-5 beta-androstane-14 beta, 17 alpha -diol;
3 beta -(2-(2-Amidino)ethoxy)-17 beta -(3-furyl)-5 beta-androstane-14 beta, 17 alpha -diol;
3 beta -(2-(2-(1-Pyrrolidinyl)ethoxy)ethoxy)-17 beta -(3-furyl)-5 beta -androstane-14 beta, 17 alpha -diol;
3 beta -(2-Guanidinoethoxy)-17 beta -(3-furyl)5 beta-androstane-14 beta, 17 alpha -diol;
3 beta -(3-Guanidinopropoxy)-17 beta -(3-furyl)-5 beta-androstane-14 beta, 17 alpha -diol;
3 beta -(3-Amino-2-hydroxypropoxy)-17 beta -(3-furyl)-5 beta-androstane-14 beta, 17 alpha -diol;
3 beta -(2,3-Diaminopropoxy)-17 beta -(3-furyl)5 beta-androstane-14 beta, 17 alpha -diol;
17 beta -(3-Furyl)-17 alpha -methoxy-5 beta -androstane-3 beta, 14 beta -diol;
17 beta -(3-Furyl)-17 alpha -(2-(1-pyrrolidinyl)ethoxy)-5 beta-androstane-3 beta, 14 beta -diol;
17 beta -(3-Furyl)-17 alpha -(3-aminopropoxy)-5 beta-androstane-3 beta, 14 beta -diol;
3 beta -(2-(1-Pyrrolidinyl)ethoxy)-17 beta -(3-furyl)-17 alpha-methoxy-5 beta -androstan-14 beta -ol;
3 beta, 17 alpha -Bis(2-(1-pyrrolidinyl)ethoxy)-17 beta -(3-furyl)-5 beta -androstan-14 beta -ol;
3 beta, 17 alpha -Bis(3-aminopropoxy)-17 beta -(3-furyl)-5 beta -androstan-14 beta -ol;
14 beta, 17 alpha -Dihydroxy-17 beta -(3-furyl)-5 beta-androstan-3-one;
3-Guanidinoimino-17 beta -(3-furyl)-5 beta -androstane-14 beta, 17 alpha -diol;
17 beta -(4-Pyridazinyl)-5 beta -androstane-3 beta, 14 beta, 17 alpha -triol;
3 beta -(2-Hydroxyethoxy)-17 beta -(4-pyridazinyl)5 beta-androstane-14 beta, 17 alpha -diol;
3 beta -(3-Aminopropoxy)-17 beta -(4-pyridazinyl)-5 beta-androstane-14 beta,17 alpha -diol;
3 beta -(2-(1-Pyrrolidinyl)ethoxy)-17 beta -(4-pyridazinyl)-5 beta -androstane-14 beta, 17 alpha -diol;
3 beta -(3-(1-Pyrrolidinyl)propoxy)-17 beta -(4-pyridazinyl)-5 beta -androstane-14 beta, 17 alpha -diol;
17 beta -(4-Pridazinyl)-17 alpha -(3-aminopropoxy)-5 beta-androstane-3 beta, 14 beta -diol;
3 beta -(2-(1-Pyrrolidinyl)ethoxy)-17 beta -(4-pyridazinyl)-17 alpha -methoxy-5 beta -androstan-14 beta -ol;
3 beta -(2-(1-Pyrrolidinyl)ethoxy)-17 beta -(4-pyridazinyl)-17 alpha -(3-amino-propoxy)-5 beta -androstan-14 beta -ol;
14 beta, 17 alpha -Dihydroxy-17 beta -(4-pyridazinyl)-5 beta-androstan-3-one;
3-Guanidinoimino-17 beta -(4-pyridazinyl)-5 beta -androstane-14 beta, 17 alpha -diol;
14 beta, 15 beta -Epoxy-17 beta -(3-furyl)-5 beta -androstane-3 beta, 17 alpha -diol;
3 beta-(2-Hydroxyethoxy)-14 beta, 15 beta -epoxy-17 beta -(3-furyl)-5 beta -androstan-17 alpha -ol;
3 beta -(3-Aminopropoxy)-14 beta, 15 beta -epoxy-17 beta -(3-furyl)-5 beta -androstan-17 alpha -ol;
3 beta -(2-(1-Pyrrolidinyl)ethoxy)-14 beta, 15 beta -epoxy-17 beta -(3-furyl)-5 beta -androstan-17 alpha -ol;
3 beta -(3-(1-Pyrrolidinyl)propoxy)-14 beta, 15 beta -epoxy-17 beta -(3-fury)-5 beta -androstan-17 alpha -ol;
3 beta -(2-(1-Pyrrolidinyl)ethoxy)-17 beta -(3-furyl)-17 alpha-methoxy-14 beta, 15 beta -epoxy-5 beta -androstane;
17 alpha -Hydroxy-17 beta -(3-furyl)-14 beta, 15 beta -epoxy-5 beta -androstan-3-one;
3-Guanidinoimino-17 beta -(3-furyl)-14 beta, 15 beta -epoxy-5 beta -androstan-17 alpha -ol;
14 beta, 15 beta -Epoxy-17 beta -(4-pyridazinyl)-5 beta-androstane-3 beta, 17 alpha -diol;
and the 3 alpha derivatives of the above identified 3 beta derivatives and also the corresponding 3 alpha and 3 beta thioderivatives where Y = S.

### BACKGROUND OF THE INVENTION

Restenosis is an obstructive lesion of the vessel that frequently occurs following surgery with mechanical angioplasty balloon (PTCA) or after endoartherectomy, performed for artery disease or organ transplantation.

This process has been considered a result of a succession of events: a) endothelial cells damage b) elastic recoil after the stretching of the artery c) neointimal hyperplasia due to proliferation and migration of vascular smooth muscle cells (SMC) d) remodelling and contraction of artery (Austin GE et al. J. Am. Coll. Cardiol. 6: 369-75, 1985).

In particular the neointimal hyperplasia leads to the re-obstruction of injured artery and is the result of platelet and leukocyte activation besides the proliferation of SMC. Pharmacological inhibition of restenosis often failed when drugs were administered by systemic delivery (Karthikeyan G and Bhargava B Curr. Opin. Cardiol. 19: 500-9. 2004).

Moreover, restenosis after angioplasty or endoartherectomy are due to damaged intima cells; angioplasty or endoartherectomy initiates a number of responses in the vessel wall including cellular migration, proliferation, and matrix accumulation, all of which contribute to neointima formation and restenosis (Malik N et al; Circulation 1998 Oct 20; 98 (16): 1657-65). Inducing apoptosis may be beneficial also to reverse vascular disease, as pulmonary vascular disease (Cowan K. N, et al. Circ. Res. 1999 May 28; 84 (10): 1223-33).

A significant improvement in the prevention of restenosis has been realized with the implantation of a polymer stent at the site of surgery (Sigwart U et al. N. Engl. J. Med. 316: 701-716, 1987). In addition, the implantation of a stent offers the opportunity to vehicle drugs locally through a slow release (Laroia ST and Laroia AT Cardiol. Rev. 12: 37-43, 2004).

The present invention also relates to 17beta-(3-furyl) and (4-pyridazinyl)-5beta, 14beta-androstane derivatives as inhibitors of organ fibrosis, including kidney fibrosis, heart fibrosis, pancreas fibrosis, lung fibrosis, vascular vessel fibrosis, bone marrow fibrosis and liver fibrosis.

Acute or chronic lesion of the organs can be developed by a variety of factors including noxious materials such as virus, chemicals and undernourishment.

Ethanol is an extremely potent hepatotoxin and can lead to cirrhosis of the liver upon prolonged exposure. In fact 20% of chronic alcoholics will eventually experience cirrhosis. The process of cirrhosis of the liver involves a series of steps beginning with fatty infiltration which leads to necrosis or cell death, then fibrosis which in turn leads to cirrhosis.

Cirrhosis is not only the terminal stage of chronic liver diseases such as viral or alcoholic hepatitis, but also progresses highly frequently to hepatocellular carcinoma. Liver fibrosis is thought to be an outcome of excess deposition of extracellular matrices such as collagen during the repair of liver tissue when the balance is lost between hepatocyte necrosis triggered by an external factor, such as a virus and alcohol, or an internal factor involving autoimmune abnormality, and liver regeneration to maintain liver functions. At the cellular level, hepatocyte disorders and necrosis activate Kupffer's cells, endothelial cells and the like, so that TNF-.alpha., TGF-.beta., and PDGF are released from the activated Kupffer's cells and endothelial cells. It is considered that those factors then activate Hepatic stellate cells as the main factor of liver fibrosis, so that cellular growth and collagen synthesis are triggered.

Even in organs such as the lungs, kidneys, heart, pancreas, and skin, similarly to the liver, it is believed that fibroblasts existing in the individual organs and stromal cells specific to the individual organs (kidney mesangial cells, pancreatic stellate cells, etc.) lapse into abnormal growth and extracellular matrix synthesis due to the stimulation by various cytokines, leading to the occurrence of organ fibrosis.

17-(3-Furyl) and (4-pyridaziny)-5 beta, 14 beta-androstane derivative are known compound.

EP0583578B1 describes the beta-androstane derivatives claimed in the present application, a process for their preparation and their use for the treatment of cardiovascular disorders such as heart failure and hypertension.

EP0590271B1 describes 17-aryl and 17-heterocyclyl-5 alpha, 14 beta -androstane, androstene and androstadiene derivatives, a process for their preparation and their use for the treatment of cardiovascular disorders such as heart failure and hypertension.

EP O590272B1 describes 17-Aryl and 17-heterocyclyl-5 beta, 14 beta-androstane derivatives and their use for the treatment of cardiovascular disorders such as heart failure and hypertension.

None of the publications above mentioned disclose the use of the 5beta, 14beta-androstane derivatives for the prevention and/or treatment of restenosis after angioplastic or endoartherectomy and organ fibrosis.

Forte et al. ("PST 2238 treatment affects gene expression in arteriotomy-injured Milan hypertensive rat strain carotids" JOURNAL OF HYPERTENSION, vol. 23, no. Suppl. 2, June 2005 (2005-06), page S244) discloses that 17beta-(3-furyl)-5beta-androstane-3beta,14beta,17alpha-triol treatment affects gene expression in arteriotomy-injured Milan hypertensive rat strain carotids and induces a reduced activation of vascular cell proliferation stimulated by arteriotomy

WO 2006/044916 A discloses the use of 17beta-(3-furyl)-5beta-androstane-3beta, 14beta, 17alpha-triol for treating a.o. restenosis, vascular restenosis, hypertrophic scars (keloid), synovitis, bacterial ulcers, abnormal wound healing, hypertrophy following surgery.

To date, no pharmaceutical agents effective as an organ fibrosis inhibitor have been sold on the market.

Therefore, the development of a pharmaceutical agent with a significant direct efficacy on organ fibrosis is needed.

It is therefore an object of the present invention a compound of formula (I), 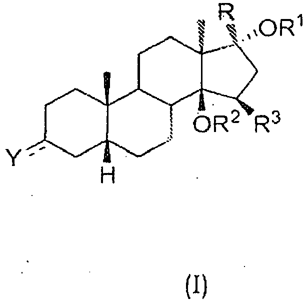 wherein the meaning of the substituents is mentioned above, for the prevention or treatment of a disease due to organ fibrosis selected from the group consisting of: kidney fibrosis; heart fibrosis; pancreas fibrosis; lung fibrosis; vascular vessel fibrosis; bone marrow fibrosis, liver fibrosis; or organs fibrosis due to systemic sclerosis.

Preferred examples of specific compounds of formula (I) are selected from the group consisting of:
17 beta -(3-Furyl)-5 beta -androstane-3 beta, 14 beta, 17 alpha -triol;
3 beta -(2-Hydroxyethoxy)-17 beta -(3-furyl)-5 beta-androstane-14 beta, 17 alpha-diol;
3 beta -(2-Aminoethoxy)-17 beta -(3-furyl)-5 beta -androstane-14 beta, 17 alpha -diol;
3 beta -(3-Aminopropoxy)-17 beta -(3-furyl)-5 beta-androstane-14 beta, 17 alpha -diol;
3 beta -(2-Methylaminoethoxy)-17 beta -(3-furyl)-5 beta-androstane-14 beta ,17 alpha -diol;
3 beta -(2-(1-Pyrrolidinyl)ethoxy)-17 beta -(3-furyl)-5 beta-androstane-14 beta, 17 alpha -diol;
3 beta -(2-(3-(1-Pyrrolidinyl)propoxy)ethoxy)-17 beta -(3-furyl)-5 beta -androstane-14 beta, 17 alpha -diol;
3 beta -(3-(1-Pyrrolidinyl)propoxy)-17 beta -(3-furyl)-5 beta-androstane-14 beta, 17 alpha-diol;
3 beta -(2-(1-Imidazolyl)ethoxy)-17 beta -(3-furyl)-5 beta-androstane-14 beta, 17 alpha -diol;
3 beta -(2-(2-Imidazolin-2-yl)ethoxy)-17 beta -(3-furyl)-5 beta-androstane-14 beta, 17 alpha -diol;
3 beta -(2-(2-Amidino)ethoxy)-17 beta -(3-furyl)-5 beta-androstane-14 beta, 17 alpha -diol;
3 beta -(2-(2-(1-Pyrrolidinyl)ethoxy)ethoxy)-17 beta -(3-furyl)-5 beta -androstane-14 beta, 17 alpha -diol;
3 beta -(2-Guanidinoethoxy)-17 beta -(3-furyl) 5 beta-androstane-14 beta, 17 alpha -diol;
3 beta -(3-Guanidinopropoxy)-17 beta -(3-furyl)-5 beta-androstane-14 beta, 17 alpha -diol;
3 beta -(3-Amino-2-hydroxypropoxy)-17 beta -(3-furyl)-5 beta-androstane-14 beta, 17 alpha -diol;
3 beta -(2,3-Diaminopropoxy)-17 beta -(3-furyl)5 beta-androstane-14 beta, 17 alpha -diol;
17 beta -(3-Furyl)-17 alpha -methoxy-5 beta -androstane-3 beta, 14 beta -diol;
17 beta -(3-Furyl)-17 alpha -(2-(1-pyrrolidinyl)ethoxy)-5 beta-androstane-3 beta, 14 beta -diol;
17 beta -(3-Furyl)-17 alpha -(3-aminopropoxy)-5 beta-androstane-3 beta, 14 beta -diol;
3 beta -(2-(1-Pyrrolidinyl)ethoxy)-17 beta -(3-furyl)-17 alpha-methoxy-5 beta -androstan-14beta -ol;
3 beta, 17 alpha -Bis(2-(1-pyrrolidinyl)ethoxy)-17 beta -(3-furl)-5 beta -androstan-14 beta -ol;
3 beta, 17 alpha -Bis(3-aminopropoxy)-17 beta -(3-furyl)-5 beta -androstan-14 beta -ol;
14 beta, 17 alpha -Dihydroxy-17 beta -(3-furyl)-5 beta-androstan-3-one;
3-Guanidinoimino-17 beta -(3-furyl)-5 beta -androstane-14 beta, 17 alpha -diol;
17 beta -(4-Pyridazinyl)-5 beta -androstane-3 beta, 14 beta, 17 alpha -triol;
3 beta -(2-Hydroxyethoxy)-17 beta -(4-pyridazinyl)5 beta-androstane-14 beta, 17 alpha -diol;
3 beta -(3-Aminopropoxy)-17 beta -(4-pyridazinyl)-5 beta-androstane-14 beta, 17 alpha -diol;
3 beta -(2-(1-Pyrrolidinyl)ethoxy)-17 beta -(4-pyridazinyl)-5 beta -androstane-14 beta, 17 alpha -diol;
3 beta -(3-(1-Pyrrolidinyl)propoxy)-17 beta -(4-pyridazinyl)-5 beta -androstane-14 beta, 17 alpha -diol;
17 beta -(4-Pridazinyl)-17 alpha -(3-aminopropoxy)-5 beta-androstane-3 beta, 14 beta -diol;
3 beta -(2-(1-Pyrrolidinyl)ethoxy)-17 beta -(4-pyridazinyl)-17 alpha -methoxy-5 beta -androstan-14 beta -ol;
3 beta -(2-(1-Pyrrolidinyl)ethoxy)-17 beta -(4-pyridazinyl)-17 alpha -(3-amino-propoxy)-5 beta -androstan-14 beta -ol;
14 beta, 17 alpha -Dihydroxy-17 beta -(4-pyridazinyl)-5 beta-androstan-3-one;
3-Guanidinoimino-17 beta -(4-pyridazinyl)-5 beta -androstane-14 beta, 17 alpha -diol;
14 beta, 15 beta -Epoxy-17 beta -(3-furyl)-5 beta -androstane-3 beta, 17 alpha -diol;
3 beta -(2-Hydroxyethoxy)-14 beta, 15 beta -epoxy-17 beta -(3-furyl)-5 beta -androstan-17 alpha -ol;
3 beta -(3-Aminopropoxy)-14 beta, 15 beta -epoxy-17 beta -(3-furyl)-5 beta -androstan-17 alpha -ol;
3 beta -(2-(1-Pyrrolidinyl)ethoxy)-14 beta, 15 beta -epoxy-17 beta -(3-furyl)-5 beta -androstan-17 alpha -ol;
3 beta -(3-(1-Pyrrolidinyl)propoxy)-14 beta, 15 beta -epoxy-17 beta -(3-furyl)-5 beta -androstan-17 alpha -ol;
3 beta -(2-(1-Pyrrolidanyl)ethoxy)-17 beta -(3-fury)-17 alpha-methoxy-14 beta, 15 beta -epoxy-5 beta -androstane;
17 alpha -Hydroxy-17 beta -(3-furyl)-14 beta, 15 beta -epoxy-5 beta -androstan-3-one;
3-Guanidinoimino-17 beta -(3-furyl)-14 beta, 15 beta -epoxy-5 beta -androstan-17 alpha -ol;
14 beta, 15 beta -Epoxy-17 beta -(4-pyridazinyl)-5 beta - androstane-3 beta, 17 alpha -diol; or the 3 alpha derivatives of the above identified 3 beta derivatives and also the corresponding 3 alpha and 3 beta thioderivatives where Y = S.

The most preferred example of specific compound according to the present invention is 17 beta -(3-Furyl)-5 beta -androstane-3 beta, 14 beta, 17 alpha -triol, in the following mentioned as "rostafuroxin".

It is a further object of the present invention the use of a compound of formula (I) wherein the meaning of the substituents is mentioned above for the manufacture of a medicament for the prevention and or treatment of a disease due to organ fibrosis selected from the grove consisting of: kidney fibrosis due to diabetic nephropathy, glomerulonephritis, or nephrosclerosis; heart fibrosis due to chronic coronary insufficiency or aging; pancreas fibrosis due to pancreatic diseases; lung fibrosis due to lung diseases; vascular vessel fibrosis due to vascular degenerative diseases such as arteriosclerosis or restenosis after vascular disobliteration following Coronary by-pass surgery and percutaneous transluminal coronary angioplasty (PTCA) or shunt insertion; bone marrow fibrosis; liver fibrosis due to virus and alcoholic chronic hepatitis, non-alcoholic steatohepatitis (NASH), cirrhosis and liver cancer; and other organs fibrosis due to systemic sclerosis.

The term "therapeutically effective amount" as used herein refers to an amount of a therapeutic agent needed to treat, ameliorate a targeted disease or condition, or to exhibit a detectable therapeutic effect.

The precise effective amount for a human subject will depend upon the severity of the disease state, general health of the subject, age, weight, and gender of the subject, diet, time and frequency of administration, drug combination (s), reaction sensitivities, and tolerance/response to therapy. This amount can be determined by routine experimentation and is within the judgement of the clinician. Generally, an effective dose per day will be from 0.05 mg to 20 mg, preferably 0.5 mg to 15 mg, most preferably 5 mg to 10 mg of a compound of formula (1), preferred is rostafuroxin.

Dosage treatment may be a single dose schedule or a multiple dose schedule, according to the physician judgement.

Compositions may be administered individually to a patient or may be administered in combination with other agents, drugs or hormones.

The medicament may also contain a pharmaceutically acceptable carrier, for administration of a therapeutic agent. Such carriers include antibodies and other polypeptides, genes and other therapeutic agents such as liposomes, provided that the carrier does not itself induce the production of antibodies harmful to the individual receiving the composition, and which may be administered without undue toxicity.

Suitable carriers may be large, slowly metabolised macromolecules such as proteins, polysaccharides, polylactic acids, polyglycolic acids, polymeric amino acids, amino acid copolymers and inactive virus particles.

A thorough discussion of pharmaceutically acceptable carriers is available in Remington's Pharmaceutical Sciences (Mack Pub. Co., N. J.1991).

Pharmaceutically acceptable carriers in therapeutic compositions may additionally contain liquids such as water, saline, glycerol and ethanol. Additionally, auxiliary substances, such as wetting or emulsifying agents, pH buffering substances, and the like, may be present in such compositions. Such carriers enable the pharmaceutical compositions to be formulated as tablets, pills, dragees, capsules, liquids, gels, syrups, slurries, suspensions, and the like, for ingestion by the patient.

Once formulated, the compositions of the invention can be administered directly to the subject. The subjects to be treated can be animals; in particular, human subjects can be treated.

The medicament of this invention may be administered by any number of routes including, but not limited to, oral, intravenous, intramuscular, intra-arterial, intramedullary, intrathecal, intraventricular, transdermal or transcutaneous applications, subcutaneous, intraperitoneal, intranasal, enteral, topical, sublingual, rectal means or locally on the diseased tissue after surgical operation. The compound of the invention may also be applied (coated) on the stent even incorporated into a controlled-release matrix.

### Discussion of the drawings

The drawings show the effect of rostafuroxin (1) in the prevention of restenosis after endoartherectomy in MHS and MNS rats (hematoxilin-orcein staining, 10x magnification); and (2) in the prevention of liver fibrosis in rats (Trichrome stain, 200X magnification).
**Figure 1(A)** cross-section of MHS carotid not injured of a rat not treated.
**Figure 1 (B)** cross-section of MHS carotid injured, harvested 30 days after surgery, of a rat treated orally with Methocel 0.5%.
**Figure 1 (C)** cross-section of MHS carotid injured, harvested 30 days after surgery, of a rat treated orally with Rostafuroxin 100 µg/kg.
**Figure 2(A)** cross-section of MNS carotid not injured of a rat not treated.
**Figure 2 (B)** cross-section of MNS carotid injured, harvested 30 days after surgery, of a rat treated orally with Methocel 0.5%.
**Figure 2 (C)** cross-section of MNS carotid injured, harvested 30 days after surgery, of a rat treated orally with Rostafuroxin 100 µg/kg.
**Figure 3** shows normal staining patterns of the liver in control rat (Ctr. group).
**Figure 4** shows that CCl4 liver injury induces remarkable hepatocellular damage which in turn stimulates the onset of a complex wound healing process, leading to hepatic fibrosis (Ctr. LF group).
**Figure 5** shows that in Rostafuroxin treated animals (RLF group), less extended tissue damage is associated to a minor degree of liver fibrosis.
**Figure 6** shows normal staining pattern of the liver in control rat (Ctr. group): score 0.
**Figure 7** shows the histological appearance of liver from two CCl4 treated rats (Crt. LF group) with fibrotic lesions of moderate degree: score 2.
**Figure 8** shows the histological appearance of liver from two CC14 rats treated with Rostafuroxin at 1mg/kg os (RLF group) with fibrotic lesions of mild degree: score 1.

The following non-limiting examples further illustrate the invention.

### EXAMPLE 1 (Reference Example)

To test the activity of the compound of the invention for the prevention of restenosis after endoartherectomy, spontaneous hypertensive MHS rats with genetic arterial hypertension (Bianchi G., Barber B. R., Torielli L., Ferrari P. The Milan hypertensive strain of rats. Genetic Model of Hypertension. 1994; 22: 457-460) were used. MHS rats are available at Prassis Research Institute, Sigmatau, Italy.

Two groups of 25-day -old MHS rats were orally treated by gavage with vehicle (Methocel 05.%) or Rostafuroxin (100 g/kg) for 6 weeks. After this period rats were subjected to the vascular surgical injury according to the protocol listed below. After surgical injury, rats were maintained under treatment either with vehicle or Rostafuroxin for further 30 days and then sacrificed for carotid artery sampling. Some post- surgery mortality was observed in the two rat strains. The final number of rats used for the histological carotid analysis was; MHS vehicle=7, MHS Rostafuroxin= 9.

### Vascular injury

The carotid surgical injury model used on MHS and MNS rat is described in J. Cell. Physiol. 2001; 186:307-313.

Briefly, a plastic Scanlon clamp for coronary artery bypass grafting was placed for 10 seconds on the carotid artery in order to cause a crushing lesion to vessel. At the same point where the clamp was applied, a 0.5 mm longitudinal incision was performed on the full thickness of the carotid artery. The incision did not cross to the other side of vessel. Hemostasis was obtained with a single adventitial 8.0-gauge polypropylene stitch. Once bleeding stopped, the carotid artery was carefully examined and blood pulsation was checked distally to the incision.

### Histological analysis

Carotid arteries were taken 30 days after injury. Rats were anaesthetized and carotids were dissected free from the surrounding tissues. Through thoracotomy, the left ventricle of the beating heart was cannulated with a blunt syringe. The syringe was held in place by a ligature in the ascending aorta. The vessels were perfused at physiological pressure first with saline until the effluent was clear, and then perfusion fixed with 4% buffered (pH 7) formaldehyde. An incision in the right atrium served as outflow tract. Before switching to formaldehyde, the descending thoracic aorta was clamped. Tissue samples were taken 20 min after perfusion. Samples were further fixed in 4% formaldehyde o.n., dehydrated and finally embedded in paraffin. Cross-sections (5µm) were stained with hematoxylin-orcein for elastic fiber analysis.

For each injured carotid, at least 60 serial cross-sections were observed under a light microscope at 20 x magnification; image screening and photography were performed using Leica IM 1000 System (Leica, Wetzlar, Germany). The sections of injured carotids showing maximal remodelling and proliferative phenomena were identified and further analyzed.

Lumen and medial areas were measured using the Leica IM 1000 software (Leica, Heerbrugg, Switzerland). The former was defined as the area enclosed by internal elastic lamina, while the latter was defined as the area enclosed between the external and internal elastic laminae. In order to reduce individual rat variability, the lumen and the medial areas of each treated carotid were normalized with respect to the contralateral uninjured carotid. For each contralateral uninjured carotid, at least 10 sections were analyzed and the average media and lumen areas were calculated.

Measurements were performed by two independent observers.

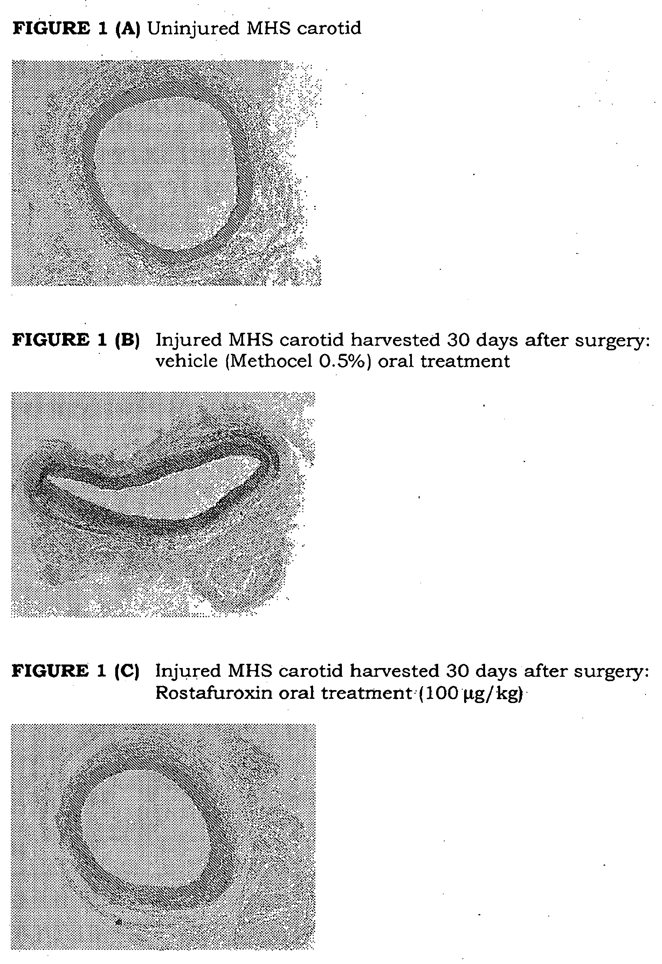

The results obtained are reported in the following Table 1 and Figure 1(A-C)

**TABLE 1**

| Morphometric measurements of the carotid lumen area of spontaneous hypertensive MHS rats. | | |
|---|---|---|
| **Animal n°** | **Injured/Uninjured carotid ratio** | |
| | **Methocel 0.5%** | **Rostafuroxin 100 µg/kg** |
| **1** | 0.65 | 0.90 |
| **2** | 0.48 | 0.89 |
| **3** | 0.41 | 0.86 |
| **4** | 0.35 | 0.83 |
| **5** | 0.31 | 0.81 |
| **6** | 0.23 | 0.66 |
| **7** | 0.22 | 0.54 |
| **8** | - | 0.36 |
| **9** | - | 0.25 |
| **mean** | 0.379 | 0.680 |
| **±SD** | 0.057 | 0.081 |
| **Student't test** | | p< 0.0094 |

The results reported in Table 1/Figure 1 (A-C) show that the compound of the invention reduced in statistically significant manner (-45%) the arterial restenosis in MHS rats with arterial hypertension.

### EXAMPLE 2 (Reference Example)

To results reported in Example 1 were confirmed using MNS rats without arterial hypertension. The method used was the same as described in Example 1.

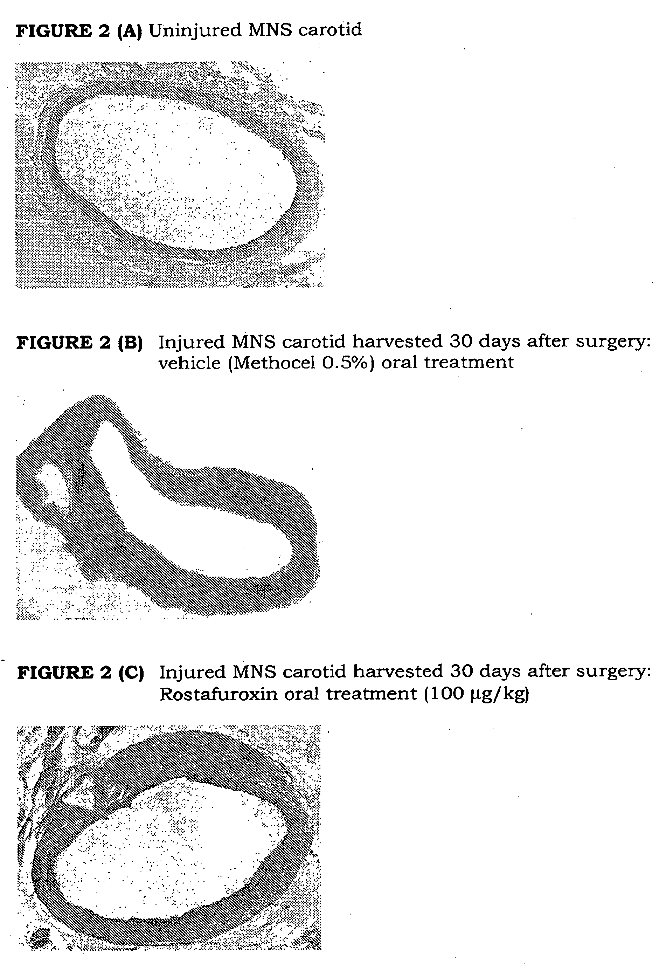

The results obtained are reported in the following Table 2 and Figure 2(A-C).

**TABLE 2**

| Morphometric measurements of the carotid lumen area of MNS rats without arterial hypertension. | | |
|---|---|---|
| | **Injured/Uninjured carotid ratio** | |
| **Animal n°** | **Methocel 0.5%** | **Rostafuroxin 100 µg/kg** |
| **1** | 0.94 | 1.00 |
| **2** | 0.80 | 0.95 |
| **3** | 0.65 | 0.93 |
| **4** | 0.60 | 0.83 |
| **5** | 0.58 | 0.80 |
| **6** | 0.56 | 0.76 |
| **7** | 0.53 | 0.75 |
| **8** | 0.51 | 0.68 |
| **9** | 0.49 | 0.59 |
| **10** | 0.47 | 0.36 |
| **11** | 0.45 | - |
| **12** | 0.38 | - |
| **13** | 0.36 | - |
| **14** | 0.24 | - |
| **Mean** | 0.540 | 0.765 |
| **±SD** | 0.047 | 0.060 |
| **Student't test** | | p< 0.0084 |

The results reported in Table 2, which are statistically significant, confirmed the results obtained in Example 1.

### EXAMPLE 3

To test the activity of the compound of the invention for the prevention and treatment of organ fibrosis, spontaneous hypertensive MHS rats with genetic arterial hypertension (Bianchi G., Barber B. R., Torielli L., Ferrari P. The Milan hypertensive strain of rats. Genetic Model of Hypertension. 1994; 22: 457-460) were used. MHS rats are available at Prassis Research Institute, Sigma-tau, Italy.

Liver fibrosis was induced by CCl₄ oral administration as described in J. Hepatol. 1999; 30: 621-631, with minor modifications: 0.375 ml/kg of CCl₄ dissolved in olive oil was administered three times per week for three weeks by oral gavage to rats starting from the 7^{th} week after weaning. Control rats received only the vehicle (olive oil). Rostafuroxin treatment started 7 weeks before the induction of liver fibrosis by CCl₄ administration (pretreatment) and was continued during the CCl₄ treatment for three weeks. Rostafuroxin was orally administered at 1mg/kg/day, suspended in Methocel (0.5%). A total of twenty four MHS rats of 25 days of age (weaning) were subdivided in three groups of 8 rats each: the first group, considered as negative control for liver fibrosis induction (Ctr.), was orally treated with the Rostafuroxin vehicle (Methocel 0.5%) along the entire period (10 weeks) and received the olive oil as vehicle of CCl₄ at the 7^{th} week after weaning for three weeks; the second group, considered as positive control for liver fibrosis (Ctr. LF), was orally treated with the Rostafuroxin vehicle (Methocel 0.5 %) along the entire period (10 weeks) and received CCl₄ at the 7^{th} week after weaning for three weeks; the third group, Rostafuroxin treated with liver fibrosis (RLF), received Rostafuroxin at 1 mg/kg along the entire period (10 weeks) and received CCl₄ at the 7^{th} week after weaning for three weeks. At the end of the treatment (10^{th} week), rats were weighed and anaesthetized with pentobarbital for blood sampling and organ removal. The blood was let clotted and serum separated after centrifugation and saved for measurement of serum albuminemia and transaminases (ALT and AST) on a Pentra 400 (ABX) Automatic Hematology Analyzer. Spleen and liver were removed and weighed. Samples of the liver median lobe were immediately frozen in liquid nitrogen and kept at -80° for further biochemical measurement of the 4-Hydroxiproline liver content, taken as an index of collagen deposition (Biochem. J. 1961; 80(1): 148-154). The rest of the liver was preserved in a 10% buffered formalin solution for the histological analysis.

### Hydroxyproline measurement

Hydroxyproline liver content was determined as described in Anal. Biochem.1981; 112: 70-75, with slight modifications.

Liver tissue (0.4 g) was homogenized in 3 ml 6 N HCl and hydrolyzed at 110 °C for 24 h. After centrifugation at 14000 rpm for 10 min, 100 µl of supernatant were neutralized with 50 µl 10N NaOH and 150 µl 1N NaHCO3. After centrifugation at 14000 for 5 min, 100 µl of supernatant were mixed with 200 µl of acetate citrate buffer plus Chloramine T pH 6.0, after incubation for 10 min at RT, 1.3 ml of Ehrlich's reagent was added and the mixture was incubated at 60 °C for 25 min. After cooling, the absorbance was measured at 558 nm (Jasco V-530).

### Histopathology and morphometry

For each case, the median and the left lobes of the liver were subjected to standard trimming and embedding procedures; slides were stained with Hematoxylin and Eosin (H&E) (Laboratory Methods in Histotechnology; Armed Forces Institute of Pathology Published by the American Registry of Pathology Washington, D.C. 1992). Special techniques were also applied in order to allow proper connective tissue detection (Masson's Trichrome stain - Laboratory Methods in Histotechnology; Armed Forces Institute of Pathology Published by the American Registry of Pathology Washington, D.C. 1992) as well as specific collagen detection and hepatic fibrosis evaluation (Syrius red staining method as described in The Journal of Histochemistry and Citochemistry 1985; vol 33, No. 8, pp. 737-743).

Liver fibrosis was qualitatively scored on Syrius red stained slides as follows:
0 = Absence of liver fibrosis, but normal staining pattern only;
1 = Mild degree, corresponding to centrilobular/periportal fibrosis, with predominantly uncomplete septal expansion - mild focal interstitial fibrosis;
2 = Moderate degree, corresponding to diffuse bridging fibrosis, with focal nodular appearance - multifocal interstitial fibrosis;
3 = Severe degree, corresponding to confluent fibrosis,
with diffuse nodular appearance - severe diffuse interstitial fibrosis.

The histopathologic findings were detected at the "ECLIPSE E800M"light microscope (Nikon Instruments S.p.A.), equipped with a 3CCD color video - camera JVC "KY F55BE", connected to a personal computer.

Relevant pictures were stored by the use of a suitable analytical software for image processing and recording (ARKON, A&P Software, Genos - Ark).

A quantitative analysis of the degree of fibrosis, following the histological qualitative analysis, was performed by digital imaging conversion of the histological pictures by a previously described method (J. Gastroenterol. 2004, Oct 1; 10(19)2894-2897).

Briefly, after staining with Sirius Red, liver collagen content was measured by histomorphometric method. A series of pictures were made on the whole area of the section (Leica DM-IRE2). Each picture was analyzed by computer system ImageJ version 1.39 for digital image analysis. Detection thresholds were set for the red color of stained collagen. The fibrotic area with positive staining were automatically selected, outlined and evaluated. Relative content of collagen was calculated as a percentage of positive staining pixels on the total number of pixels of the picture.

### RESULTS

The results obtained are reported in the following Tables 3-6.

Table 3 shows the body and organ weights, expressed as an index of body weight, in the three groups.

**TABLE 3**

| **Rat** | **Ctr. Methocel 0.5%** | | **Ctr. LF Methocel 0.5%** | | **RLF 1 mg/kg os** | |
|---|---|---|---|---|---|---|
| **N°** | **BW g.** | **Spleen/bw (%)** | **BW g.** | **Spleen/bw (%)** | **BW g.** | **Spleen/bw (%)** |
| **1** | 472 | 0.168 | 360 | 0.295 | 344 | 0.262 |
| **2** | 472 | 0.180 | 395 | 0.269 | 333 | 0.247 |
| **3** | 445 | 0.175 | 327 | 0.281 | 381 | 0.302 |
| **4** | 460 | 0.183 | 329 | 0.495 | 345 | 0.348 |
| **5** | 460 | 0.185 | 344 | 0.351 | 344 | 0.288 |
| **6** | 458 | 0.180 | 353 | 0.403 | 347 | 0.251 |
| **7** | 425 | 0.189 | 338 | 0.389 | 364 | 0.316 |
| **8** | dead | - | dead | - | 366 | 0.261 |
| | | | | | | |
| **Mean** | 456 | 0.180 | 349.4 | 0.355 | 353 | 0.284 |
| **sem** | 5.8 | 0.0024 | 8.3 | 0.029 | 5.6 | 0.013 |
| **p** | | | <0.01 Vs. Ctr. | <0.01 Vs. Ctr. | <0.01 vs. Ctr. | < 0.05 vs Ctr LF |

The results reported in Table 3 show that the compound of the invention reduced in statistically significant manner by 20% the spleen weight in rats with CCl₄-induced liver fibrosis.

The weight of the liver and transaminases were not modified after the treatment of the compound of the invention.

Table 4 shows the plasma albuminemia in the three groups.

**TABLE 4**

| **Rat** | **Ctr. Methocel 0.5%** | **Ctr. LF Methocel 0.5%** | **RLF 1 mg/kg os** |
|---|---|---|---|
| **N°** | Albumin g/dl | Albumin g/dl | Albumin g/dl |
| **1** | 3.50 | 2.51 | 2.95 |
| **2** | 3.57 | 2.93 | 3.19 |
| **3** | 3.46 | 3.28 | 3.07 |
| **4** | 3.52 | 3.09 | 3.82 |
| **5** | 3.63 | 2.93 | 4.04 |
| **6** | 3.40 | 3.39 | 3.65 |
| **7** | 3.62 | 3.02 | 3.36 |
| **8** | dead | dead | 4.15 |
| **Mean** | 3.53 | 3.02 | 3.53 |
| **sem** | 0.032 | 0.107 | 0.16 |
| **p** | | <0.05 Vs Ctr | <0.05 Vs Ctr LF |

The results reported in Table 4 show that the compound of the invention increased in statistically significant manner by 16.8 % the plasma levels of albumin in rats with CCl₄-induced liver fibrosis.

Table 5 shows the liver hydroxyproline content in the three groups.

**TABLE 5**

| **Rat** | **Ctr. Methocel 0.5%** | **Ctr. LF Methocel 0.5%** | **RLF 1 mg/kg os** |
|---|---|---|---|
| **N°** | hydroxyproline µg/g prot | hydroxyproline µg/g prot | hydroxyproline µg/g prot |
| **1** | 391.8 | 1353.3 | 799.8 |
| **2** | 574.0 | 2254.6 | 2762.3 |
| **3** | 538.5 | 1568.7 | 750.0 |
| **4** | 427.2 | 1564.2 | 1460.7 |
| **5** | 512.7 | 1195.4 | 886.5 |
| **6** | 467.6 | 2393.4 | 791.6 |
| **7** | 656.3 | 845.6 | 1217.4 |
| **8** | dead | dead | 811.0 |
| **Mean** | 509.72 | 1596.45 | 1184.9 |
| **sem** | 34.13 | 210.09 | 242.2 |

The results reported in Table 5 show that the compound of the invention reduced in a significant manner (25.8 %) the liver content of hydroxyproline, which is an index of collagen deposition, thus fibrosis, in rats with CCl₄-induced liver fibrosis.

### HISTOPATHOLOGY AND MORPHOMETRY

The main pathology in CCl₄ treated animals (Ctr.LF group) was represented by remarkable hepatocellular damage (ballooning degeneration and apoptosis, intracytoplasmic inclusion bodies, fatty change), chronic inflammatory reaction, extracellular matrix deposition and regenerative changes (proliferation and hyperplasia of parenchymal and non-parenchymal cells).

A minor extension of tissue damage was shown from rats treated with the Rostafuroxin.

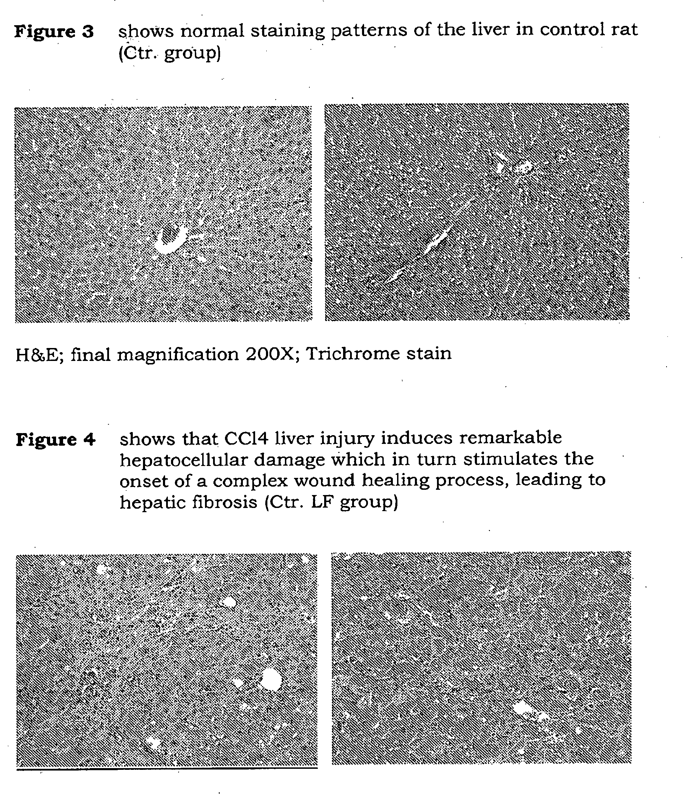

Images of the results obtained are reported in Figures 3-5.

Table 6 shows the qualitative evaluation of liver fibrosis, on the basis of the adopted scoring system on Syrius red stained slides

**TABLE 6**

| **Liver fibrosis** | **Score** | **Ctr. Methocel 0.5%** | | **Ctr. LF Methocel 0.5%** | | **RLF 1mg/kg OS** | |
|---|---|---|---|---|---|---|---|
| | | No. | % | No. | % | No. | % |
| **Absence** | 0 | 7/7 | 100 | 0/7 | 0 | 0/8 | 0 |
| **Mild** | 1 | 0/7 | 0 | 0/7 | 0 | 6/8 | 75 |
| **Moderate** | 2 | 0/7 | 0 | 6/7 | 85.7 | 2/8 | 25 |
| **Severe** | 3 | 0/7 | 0 | 1/7 | 14.3 | 0/8 | 0 |

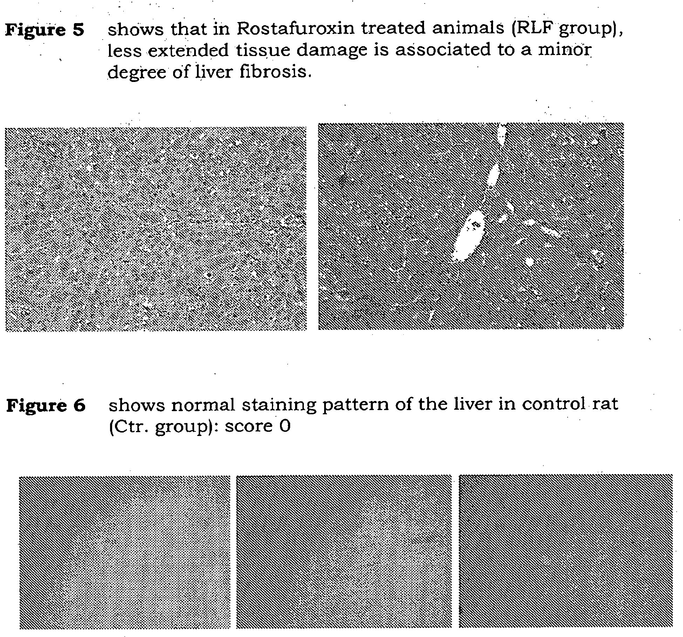

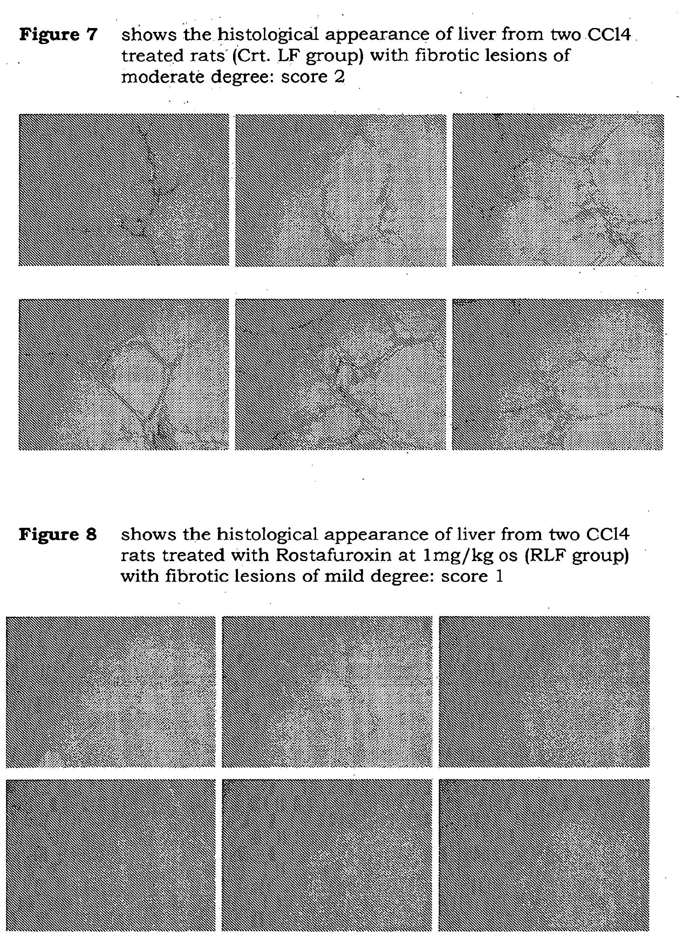

The results reported in Table 6 and figures 6-8 indicate that all animals treated with CCl₄ alone (Ctr. LF group) showed high score values of liver fibrosis, ranging from moderate (85,7% of cases) to severe in degree (one case, corresponding to 14,3%).

Conversely, mild degree of liver fibrosis was detected only in CCl4 + Rostafuroxin, the compound of the invention, treated animals (RLF group), and it was observed in the large majority of cases (75%). In the same group, a moderate degree of liver fibrosis was observed in two animals only (25%, compared to the 85,7% of CCl₄ group). Finally, no rats treated with CCl₄ + Rostafuroxin showed severe liver fibrosis.

Table 7 shows the quantitative analysis of the degree of liver fibrosis as observed by the analysis in the three groups.

**Table 7**

| | **% FIBROTIC AREA** | | |
|---|---|---|---|
| **Rat n°** | **Ctr. Methocel 0.5%** | **Ctr. LF Methocel 0.5%** | **RLF 1 mg/kg os** |
| **1** | 1.43 | 11.55 | 6.63 |
| **2** | 0.57 | 10.40 | 6.89 |
| **3** | 1.74 | 8.42 | 5.08 |
| **4** | 0.61 | 17.18 | 11.80 |
| **5** | 0.28 | 10.26 | 2.01 |
| **6** | 0.52 | 6.97 | 4.64 |
| **7** | 1.47 | 8.57 | 5.50 |
| **8** | | | 1.44 |
| | | | |
| **Mean** | 0.95 | 10.48 | 5.50 |
| **sem** | 0.22 | 1.26 | 1.14 |
| **p** | | <0.01 Vs Ctr | <0.05 Vs Ctr LF |

The results reported in Table 7 show that the compound of the invention reduced in a statistically significant manner by 47.5 % the liver fibrotic area in rats with CCl4-induced liver fibrosis.

## Claims

1. Compound of formula (I) 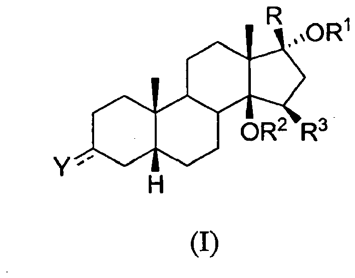 wherein:
the symbol - - - represents a single or a double bond;
Y is oxygen or guanidinoimino when - - - in position 3 is a double bond;
Y is hydroxy, OR⁴ or SR⁴, when - - - in position 3 is a single bond and can have an alpha or beta configuration;
R is an unsubstituted or substituted 3-furyl or 4-pyridazinyl group;
R¹ is hydrogen; methyl; ethyl or n-propyl substituted by OH or NR⁵R⁶;
R² is hydrogen or together to R³ is a bond of an oxirane ring;
R³ is hydrogen or together to R² is a bond of an oxirane ring;
R⁴ is hydrogen; methyl; C2-C6 alkyl or C3-C6 alkenyl or C2-C6 acyl, these alkyl, alkenyl and acyl groups being unsubstituted or substituted by a quaternary ammonium group or one or more OR⁷, NR⁸R⁹, formyl, amidino, guanidinoimino or by NR⁸R⁹ and hydroxy;
R⁵, R⁶ are independently hydrogen; methyl; C2-C6 alkyl unsubstituted or substituted by one NR¹⁰R¹¹, or NR¹⁰R¹¹ and hydroxy, or R⁵ and R⁶ taken together with the nitrogen atom form an unsubstituted or substituted saturated or unsaturated penta- or hexa-monoheterocyclic ring, optionally containing another heteroatom chosen from oxygen or sulfur or nitrogen;
R⁷ is hydrogen, methyl or C2-C4 alkyl, this alkyl being unsubstituted or substituted by one or more NR¹⁰R¹¹ or by NR¹⁰R¹¹ and hydroxy;
R⁸, R⁹ are independently hydrogen; methyl; C2-C6 alkyl or C3-C6 alkenyl, these alkyl and alkenyl groups being unsubstituted or substituted by one or more NR¹⁰R¹¹, or NR¹⁰R¹¹ and hydroxy, or R⁸ and R⁹ taken together with the nitrogen atom form an unsubstituted or substituted saturated or unsaturated penta- or hexa-monoheterocyclic ring, optionally containing another heteroatom chosen from oxygen or sulfur or nitrogen, or R⁸ is hydrogen and R⁹ is amidino; or NR⁸R⁹ represents propargylamino,
R¹⁰, R¹¹ are independently hydrogen, C1-C6 alkyl, or R¹⁰ and R¹¹, taken together with the nitrogen atom form a saturated or unsaturated penta- or hexa-monoheterocyclic ring; for the prevention or treatment of a disease due to organ fibrosis selected from the group consisting of: kidney fibrosis; heart fibrosis; pancreas fibrosis; lung fibrosis; vascular vessel fibrosis; bone marrow fibrosis, liver fibrosis; or organ fibrosis due to systemic sclerosis.

2. Compound for use according to claim 1, selected from the group consisting of:
17 beta -(3-Furyl)-5 beta -androstane-3 beta, 14 beta, 17 alpha -triol;
3 beta -(2-Hydroxyethoxy)- 17 beta -(3-furyl)-5 beta - androstane- 14 beta, 17 alpha -diol;
3 beta -(2-Aminoethoxy)- 17 beta -(3-furyl)-5 beta - androstane- 14 beta, 17 alpha -diol;
3 beta -(3-Aminopropoxy)- 17 beta -(3-furyl)-5 beta - androstane- 14 beta, 17 alpha-diol;
3 beta -(2-Methylaminoethoxy)- 17 beta -(3-furyl)-5 beta - androstane- 14 beta, 17 alpha -diol;
3 beta -(2-(1-Pyrrolidinyl)ethoxy)- 17 beta -(3-furyl)-5 beta -androstane- 14 beta, 17 alpha -diol;
3 beta -(2-(3-(1-Pyrrolidinyl)propoxy)ethoxy)- 17 beta -(3- furyl)-5 beta - androstane- 14 beta, 17 alpha -diol;
3 beta -(3-(1-Pyrrolidinyl)propoxy)- 17 beta -(3-furyl)-5 beta -androstane-14 beta, 17 alpha-diol;
3 beta -(2-(1-Imidazolyl)ethoxy)- 17 beta -(3-furyl)-5 beta - androstane- 14 beta, 17 alpha -diol;
3 beta -(2-(2-Imidazolin-2-yl)ethoxy)- 17 beta -(3-furyl)-5 beta -androstane-14 beta, 17 alpha -diol;
3 beta -(2-(2-Amidino)ethoxy)- 17 beta -(3-furyl)-5 beta - androstane- 14 beta, 17 alpha -diol;
3 beta -(2-(2-(1-Pyrrolidinyl)ethoxy)ethoxy)- 17 beta -(3- furyl)-5 beta - androstane- 14 beta, 17 alpha -diol;
3 beta -(2-Guanidinoethoxy)- 17 beta -(3-furyl)5 beta - androstane- 14 beta, 17 alpha -diol;
3 beta -(3-Guanidinopropoxy)- 17 beta -(3-furyl)-5 beta - androstane- 14 beta, 17 alpha -diol;
3 beta -(3-Amino-2-hydroxypropoxy)- 17 beta -(3-furyl)-5 beta - androstane- 14 beta, 17 alpha -diol;
3 beta -(2,3-Diaminopropoxy)- 17 beta -(3-furyl)5 beta - androstane- 14 beta, 17 alpha -diol;
17 beta -(3-Furyl)- 17 alpha -methoxy-5 beta -androstane- 3 beta, 14 beta - diol;
17 beta -(3-Furyl)- 17 alpha -(2-(1-pyrrolidinyl)ethoxy)-5 beta -androstane-3 beta, 14 beta -diol;
17 beta -(3-Furyl)- 17 alpha -(3-aminopropoxy)-5 beta - androstane-3 beta, 14 beta -diol;
3 beta -(2-(1-Pyrrolidinyl)ethoxy)- 17 beta -(3-furyl)- 17 alpha -methoxy-5 beta -androstan- 14 beta -ol;
3 beta, 17 alpha -Bis(2-(1-pyrrolidinyl)ethoxy)- 17 beta - (3-furyl)-5 beta - androstan- 14 beta -ol;
3 beta, 17 alpha -Bis(3-aminopropoxy)- 17 beta -(3-furyl)- 5 beta - androstan- 14 beta -ol;
14 beta, 17 alpha -Dihydroxy- 17 beta -(3-furyl)-5 beta - androstan-3-one; 3-Guanidinoimino- 17 beta -(3-furyl)-5 beta -androstane- 14 beta, 17 alpha-diol;
17 beta -(4-Pyridazinyl)-5 beta -androstane-3 beta, 14 beta, 17 alpha - triol;
3 beta -(2-Hydroxyethyoxy)- 17 beta -(4-pyridazinyl)5 beta - androstane-14 beta, 17 alpha -diol;
3 beta -(3-Aminopropoxy)- 17 beta -(4-pyridazinyl)-5 beta -androstane- 14 beta, 17 alpha -diol;
3 beta -(2-( 1-Pyrrolidinyl)ethoxy)- 17 beta -(4-pyridazinyl)- 5 beta-androstane- 14 beta, 17 alpha -diol;
3 beta -(3-( 1-Pyrrolidinyl)propoxy)- 17 beta -(4- pyridazinyl)-5 beta-androstane- 14 beta, 17 alpha -diol;
17 beta -(4-Pyridazinyl)- 17 alpha -(3-aminopropoxy)-5 beta -androstane-3 beta, 14 beta -diol;
3 beta -(2-( 1-Pyrrolidinyl)ethoxy)- 17 beta -(4-pyridazinyl)- 17 alpha-methoxy-5 beta -androstan- 14 beta -ol;
3 beta -(2-( 1-Pyrrolidinyl)ethoxy)- 17 beta -(4-pyridazinyl)- 17 alpha -(3-amino-propoxy)-5 beta -androstan- 14 beta -ol;
14 beta, 17 alpha -Dihydroxy- 17 beta -(4-pyridazinyl)-5 beta -androstan-3-one;
3-Guanidinoimino- 17 beta -(4-pyridazinyl)-5 beta - androstane- 14 beta, 17 alpha -diol;
14 beta, 15 beta -Epoxy- 17 beta -(3-furyl)-5 beta - androstane-3 beta, 17 alpha -diol;
3 beta -(2-Hydroxyethoxy)- 14 beta, 15 beta -epoxy- 17 beta -(3-furyl)-5 beta -androstan- 17 alpha -ol;
3 beta -(3-Aminopropoxy)- 14 beta, 15 beta -epoxy- 17 beta -(3-furyl)-5 beta -androstan- 17 alpha -ol;
3 beta -(2-( 1-Pyrrolidinyl)ethoxy)- 14 beta, 15 beta -epoxy- 17 beta -(3-furyl)-5 beta -androstan- 17 alpha -ol;
3 beta -(3-( 1-Pyrrolidinyl)propoxy)- 14 beta, 15 beta - epoxy- 17 beta -(3-furyl)-5 beta -androstan- 17 alpha -ol;
3 beta -(2-( 1-Pyrrolidinyl)ethoxy)- 17 beta -(3-furyl)- 17 alpha -methoxy-14 beta, 15 beta -epoxy-5 beta -androstane;
17 alpha -Hydroxy- 17 beta -(3-furyl)- 14 beta, 15 beta - epoxy-5 beta-androstan-3-one;
3-Guanidinoimino- 17 beta -(3-furyl)- 14 beta, 15 beta - epoxy-5 beta - androstan- 17 alpha -ol;
14 beta, 15 beta -Epoxy- 17 beta -(4-pyridazinyl)-5 beta - androstane-3 beta, 17 alpha -diol;
and the 3 alpha derivatives of the above identified 3 beta derivatives and also the corresponding 3 alpha and 3 beta thioderivatives where Y = S.

3. Use of a compound as described in claim 1 for the manufacture of a medicament for the prevention and/or treatment of a disease due to organ fibrosis selected from the group consisting of: kidney fibrosis; heart fibrosis; pancreas fibrosis; lung fibrosis; vascular vessel fibrosis; bone marrow fibrosis, liver fibrosis; or organ fibrosis due to systemic sclerosis.

4. Use according to claim 3, in which the preferred compound is 17 beta -(3-Furyl)-5 beta -androstane-3 beta, 14 beta, 17 alpha - triol.

5. Use according to claim 3, in which liver fibrosis is due to a virus disease, alcoholic hepatitis, non-alcoholic steatohepatitis, cirrhosis or liver cancer.

6. Use according to claim 3, in which the compound is administered in a dose of from 0.05 mg to 20 mg per day.

7. Use according to claim 3, in which the compound is administered in a dose of from 0.5 mg to 15 mg. 12.

8. Use according to claim 3, in which the compound is administered in a dose of from 5 mg to 10 mg.

9. Use according to claims 3, in which the compound is administered in a single dose schedule.

10. Use according to claims 3, in which the compound is administered in a multiple dose schedule.

11. Use according to claim 3, in which the composition is for oral, intravenous, intramuscular, intra-arterial, intramedullary, intrathecal, intraventricular, transdermal, transcutaneous, subcutaneous, intraperitoneal, intranasal, enteral, topical, sublingual or rectal administration.

12. Use according to claim 3, in which the composition is administered locally on the diseased tissue after surgical operation.

13. Use according to claim 3, in which the composition is coated on the stent; incorporated into a controlled-release matrix; or incorporated into liposomes.

## Patentansprüche

1. Verbindung der Formel (I) 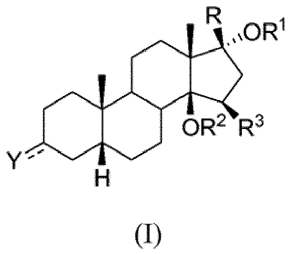 worin:
das Symbol - - - eine Einfach- oder eine Doppelbindung repräsentiert;
Y Sauerstoff oder Guanidinoimino ist, wenn - - - in Position 3 eine Doppelbindung ist;
Y Hydroxy, OR⁴ oder SR⁴ ist, wenn - - - in Position 3 eine Einfachbindung ist, und Alpha- oder Beta-Konfiguration aufweisen kann;
R eine unsubstituierte oder substituierte 3-Furyl- oder 4-Pyridazinylgruppe ist;
R¹ Wasserstoff; Methyl; Ethyl oder n-Propyl ist, substituiert mit OH oder NR⁵R⁶;
R² Wasserstoff ist oder gemeinsam mit R³ eine Bindung eines Oxiranrings ist;
R³ Wasserstoff ist oder gemeinsam mit R² eine Bindung eines Oxiranrings ist;
R⁴ Wasserstoff; Methyl; C₂-C₆-Alkyl oder C₃-C₆-Alkenyl oder C₂-C₆-Acyl ist, wobei diese Alkyl-, Alkenyl- und Acylgruppen unsubstituiert oder mit einer quartären Ammoniumgruppe oder einem oder mehreren OR⁷, NR⁸R⁹, Formyl, Amidino, Guanidinoimino oder mit NR⁸R⁹ und Hydroxy substituiert sind;
R⁵,R⁶ unabhängig voneinander Wasserstoff; Methyl; C₂-C₆-Alkyl sind, unsubstituiert oder mit einem NR¹⁰R¹¹ oder NR¹⁰R¹¹ und Hydroxy substituiert, oder R⁵ und R⁶ zusammen mit dem Stickstoffatom einen unsubstituierten oder substituierten gesättigten oder ungesättigten monoheterocyclischen Fünf- oder Sechsring bilden, der optional ein weiteres Heteroatom enthält, ausgewählt aus Sauerstoff oder Schwefel oder Stickstoff;
R⁷ Wasserstoff, Methyl oder C₂-C₄-Alkyl ist, wobei dieses Alkyl unsubstituiert oder mit einem oder mehreren NR¹⁰R¹¹ oder mit NR¹⁰R¹¹ und Hydroxy substituiert ist;
R⁸,R⁹ unabhängig voneinander Wasserstoff; Methyl; C₂-C₆-Alkyl oder C₃-C₆-Alkenyl sind, wobei diese Alkyl- und Alkenylgruppen unsubstituiert oder mit einem oder mehreren NR¹⁰R¹¹ oder NR¹⁰R¹¹ und Hydroxy substituiert sind, oder R⁸ und R⁹ zusammen mit dem Stickstoffatom einen unsubstituierten oder substituierten gesättigten oder ungesättigten monoheterocyclischen Fünf- oder Sechsring bilden, der optional ein weiteres Heteroatom enthält, ausgewählt aus Sauerstoff oder Schwefel oder Stickstoff; oder R⁸ ist Wasserstoff und R⁹ ist Amidino; oder NR⁸R⁹ repräsentiert Propargylamino, R¹⁰,R¹¹ unabhängig voneinander Wasserstoff, C₁-C₆-Alkyl sind, oder R¹⁰ und R¹¹ zusammen mit dem Stickstoffatom einen gesättigten oder ungesättigten monoheterocyclischen Fünf- oder Sechsring bilden; zur Prävention oder Behandlung einer Krankheit, die durch Organfibrose bedingt ist, ausgewählt aus der Gruppe, bestehend aus: Nierenfibrose; Herzfibrose; Pankreasfibrose; Lungenfibrose; Gefäßfibrose; Knochenmarkfibrose, Leberfibrose oder Organfibrosen aufgrund systemischer Sklerose.

2. Verbindung zur Verwendung gemäß Anspruch 1, ausgewählt aus der Gruppe, bestehend aus:
17-beta-(3-Furyl)-5-beta-androstan-3-beta,14-beta,17-alpha-triol;
3-beta-(2-Hydroxyethoxy)-17-beta-(3-furyl)-5-beta-androstan-14-beta,17-alpha-diol;
3-beta-(2-Aminoethoxy)-17-beta-(3-furyl)-5-beta-androstan-14-beta,17-alpha-diol;
3-beta-(3-Aminopropoxy)-17-beta-(3-furyl)-5-beta-androstan-14-beta,17-alpha-diol;
3-beta-(2-Methylaminoethoxy)-17-beta-(3-furyl)-5-beta-androstan-14-beta, 17-alpha-diol;
3-beta-(2-(1-Pyrrolidinyl)ethoxy)-17-beta-(3-furyl)-5-beta-androstan-14-beta, 17-alpha-diol;
3-beta-(2-(3-(1-Pyrrolidinyl)propoxy)ethoxy)-17-beta-(3-furyl)-5 beta-androstan-14-beta,17-alpha-diol;
3-beta-(3-(1-Pyrrolidinyl)propoxy)-17-beta-(3-furyl)-5-beta-androstan-14-beta, 17-alpha-diol;
3-beta-(2-(1-Imidazolyl)ethoxy)-17-beta-(3-furyl)-5-beta-androstan-14-beta,17-alpha-diol;
3-beta-(2-(2-Imidazolin-2-yl)ethoxy)-17-beta-(3-furyl)-5-beta-androstan-14-beta,17-alpha-diol;
3-beta-(2-(2-Amidino)ethoxy)-17-beta-(3-furyl)-5-beta-androstan-14-beta,17-alpha-diol;
3-beta-(2-(2-(1-Pyrrolidinyl)ethoxy)ethoxy)-17-beta-(3-furyl)-5-beta-androstan-14-beta,17-alpha-diol;
3-beta-(2-Guanidinoethoxy)-17-beta-(3-furyl)5-beta-androstan-14-beta,17-alpha-diol;
3-beta-(3-Guanidinopropoxy)-17-beta-(3-furyl)-5-beta-androstan-14-beta,17-alpha-diol;
3-beta-(3-Amino-2-hydroxypropoxy)-17-beta-(3-furyl)-5-beta-androstan-14-beta,17-alpha-diol;
3-beta-(2,3-Diaminopropoxy)-17-beta-(3-furyl)-5-beta-androstan-14-beta,17-alpha-diol;
17-beta-(3-Furyl)-17-alpha-methoxy-5-beta-androstan-3-beta,14-beta-diol;
17-beta-(3-Furyl)-17-alpha-(2-(1-pyrrolidinyl)ethoxy)-5-beta-androstan-3-beta,14-beta-diol;
17-beta-(3-Furyl)-17-alpha-(3-aminopropoxy)-5-beta-androstan-3-beta,14-beta-diol;
3-beta-(2-(1-Pyrrolidinyl)ethoxy)-17-beta-(3-furyl)-17-alpha-methoxy-5-beta-androstan-14-beta-ol;
3-beta,17-alpha-Bis(2-(1-pyrrolidinyl)ethoxy)-17-beta-(3-furyl)-5-beta-androstan-14-beta-ol;
3-beta,17-alpha-Bis(3-aminopropoxy)-17-beta-(3-furyl)-5-beta-androstan-14-beta-ol;
14-beta,17-alpha-Dihydroxy-17-beta-(3-furyl)-5-beta-androstan-3-on;
3-Guanidinoimino-17-beta-(3-furyl)-5-beta-androstan-14-beta,17-alpha-diol;
17-beta-(4-Pyridazinyl)-5-beta-androstan-3-beta,14-beta,17-alpha-triol;
3-beta-(2-Hydroxyethoxy)-17-beta-(4-pyridazinyl)5-beta-androstan-14-beta,17-alpha-diol;
3-beta-(3-Aminopropoxy)-17-beta-(4-pyridazinyl)-5-beta-androstan-14-beta, 17-alpha-diol;
3-beta-(2-(1-Pyrrolidinyl)ethoxy)-17-beta-(4-pyridazinyl)-5-beta-androstan-14-beta,17-alpha-diol;
3-beta-(3-(1-Pyrrolidinyl)propoxy)-17-beta-(4-pyridazinyl)-5-beta-androstan-14-beta,17-alpha-diol;
17-beta-(4-Pyridazinyl)-17-alpha-(3-aminopropoxy)-5-beta-androstan-3-beta, 14-beta-diol;
3-beta-(2-(1-Pyrrolidinyl)ethoxy)-17-beta-(4-pyridazinyl)-17-alpha-methoxy-5-beta-androstan-14-beta-ol;
3-beta-(2-(1-Pyrrolidinyl)ethoxy)-17-beta-(4-pyridazinyl)-17-alpha-(3-amino-propoxy)-5-beta-androstan-14-beta-ol;
14-beta,17-alpha-Dihydroxy-17-beta-(4-pyridazinyl)-5-beta-androstan-3-on;
3-Guanidinoimino-17-beta-(4-pyridazinyl)-5-beta-androstan-14-beta,17-alpha-diol;
14-beta,15-beta-Epoxy-17-beta-(3-furyl)-5-beta-androstan-3-beta,17-alpha-diol;
3-beta-(2-Hydroxyethoxy)-14-beta,15-beta-epoxy-17-beta-(3-furyl)-5-beta-androstan-17-alpha-ol;
3-beta-(3-Aminopropoxy)-14-beta,15-beta-epoxy-17-beta-(3-furyl)-5-beta-androstan-17-alpha-ol;
3-beta-(2-(1-Pyrrolidinyl)ethoxy)-14-beta,15-beta-epoxy-17-beta-(3-furyl)-5-beta-androstan-17-alpha-ol;
3-beta-(3-(1-Pyrrolidinyl)propoxy)-14-beta,15-beta-epoxy-17-beta-(3-furyl)-5-beta-androstan-17-alpha-ol;
3-beta-(2-(1-Pyrrolidinyl)ethoxy)-17-beta-(3-furyl)-17-alpha-methoxy-14-beta,15-beta-epoxy-5-beta-androstan;
17-alpha-Hydroxy-17-beta-(3-furyl)-14-beta,15-beta-epoxy-5-beta-androstan-3-on;
3-Guanidinoimino-17-beta-(3-furyl)-14-beta,15-beta-epoxy-5-beta-androstan-17-alpha-ol;
14-beta,15-beta-Epoxy-17-beta-(4-pyridazinyl)-5-beta-androstan-3-beta,17-alpha-diol;
und die 3-alpha-Derivate der oben bezeichneten 3-beta-Derivate, und auch die korrespondierenden 3-alpha- und 3-beta-Thioderivate, bei denen Y = S ist.

3. Verwendung einer Verbindung wie in Anspruch 1 beschrieben, zur Herstellung eines Medikaments zur Prävention und/oder Behandlung einer Krankheit, bedingt durch Organfibrose, ausgewählt aus der Gruppe, bestehend aus: Nierenfibrose; Herzfibrose; Pankreasfibrose; Lungenfibrose; Gefäßfibrose; Knochenmarkfibrose, Leberfibrose oder Organfibrosen aufgrund systemischer Sklerose.

4. Verwendung, gemäß Anspruch 3, bei welcher die bevorzugte Verbindung 17-beta-(3-Furyl)-5-beta-androstan-3-beta,14-beta,17-alpha-triol ist.

5. Verwendung, gemäß Anspruch 3, bei welcher die Leberfibrose durch eine Viruskrankheit, alkoholische Hepatitis, nicht-alkoholische Steatohepatitis, Zirrhose oder Leberkrebs bedingt ist.

6. Verwendung, gemäß Anspruch 3, bei welcher die Verbindung in einer Dosis von 0,05 mg bis 20 mg pro Tag verabreicht wird.

7. Verwendung, gemäß Anspruch 3, bei welcher die Verbindung in einer Dosis von 0,5 mg bis 15 mg verabreicht wird.

8. Verwendung, gemäß Anspruch 3, bei welcher die Verbindung in einer Dosis von 5 mg bis 10 mg verabreicht wird.

9. Verwendung, gemäß Anspruch 3, bei welcher die Verbindung nach einem Einzeldosisschema verabreicht wird.

10. Verwendung, gemäß Anspruch 3, bei welcher die Verbindung nach einem Merhrfachdosisschema verabreicht wird.

11. Verwendung, gemäß Anspruch 3, bei welcher die Zusammensetzung zur oralen, intravenösen, intramuskulären, intraarteriellen, intramedullären, intrathekalen, intraventrikulären, transdermalen, transkutanen, subkutanen, intraperitonealen, intranasalen, enteralen, topischen, sublingualen oder rektalen Verabreichung bestimmt ist.

12. Verwendung, gemäß Anspruch 3, bei welcher die Zusammensetzung lokal in das erkrankte Gewebe nach einem chirurgischen Eingriff verabreicht wird.

13. Verwendung, gemäß Anspruch 3, bei welcher die Zusammensetzung auf einem Stent aufgebracht ist, in eine Matrix zur kontrollierten Freisetzung eingeschlossen ist oder in Liposomen eingeschlossen ist.

## Revendications

1. Composé de formule (I) 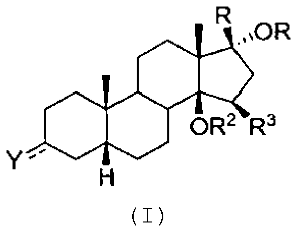 dans lequel :
le symbole - - - représente une simple ou double liaison ;
Y est oxygène ou guanidinoimino lorsque - - - en position 3 est une double liaison ;
Y est hydroxy, OR⁴ ou SR⁴, lorsque - - - en position 3 est une simple liaison et peut avoir une configuration alpha ou bêta ;
R est un groupe 3-furyle ou 4-pyridazinyle non substitué ou substitué ;
R¹ est hydrogène ; méthyle ; éthyle ou n-propyle substitué par OH ou NR⁵R⁶ ;
R² est hydrogène ou conjointement avec R³ est une liaison d'un cycle oxirane ;
R³ est hydrogène ou conjointement avec R² est une liaison d'un cycle oxirane ;
R⁴ est hydrogène ; méthyle ; alkyle en C2-C6 ou alcényle en C3-C6 ou acyle en C2-C6, ces groupes alkyle, alcényle et acyle étant non substitués ou substitués par un groupe ammonium quaternaire ou un ou plusieurs OR⁷, NR⁸R⁹, formyle, amidino, guanidinoimino ou par NR⁸R⁹ et hydroxy ;
R⁵, R⁶ sont indépendamment hydrogène ; méthyle ; alkyle en C2-C6 non substitué ou substitué par un NR¹⁰R¹¹, ou NR¹⁰R¹¹ et hydroxy, ou R⁵ et R⁶ conjointement avec l'atome d'azote forment un cycle penta- ou hexa-monohétérocyclique saturé ou insaturé, non substitué ou substitué, contenant facultativement un autre hétéroatome choisi parmi l'oxygène ou le soufre ou l'azote ;
R⁷ est hydrogène, méthyle ou alkyle en C2-C4, cet alkyle étant non substitué ou substitué par un ou plusieurs NR¹⁰R¹¹ ou par NR¹⁰R¹¹ et hydroxy ;
R⁸, R⁹ sont indépendamment hydrogène ; méthyl ; alkyle en C2-C6 ou alcényle en C3-C6, ces groupes alkyle et alcényle étant non substitués ou substitués par un ou plusieurs NR¹⁰R¹¹, ou NR¹⁰R¹¹ et hydroxy, ou R⁸ et R⁹ conjointement avec l'atome d'azote forment un cycle penta- ou hexa-monohétérocyclique saturé ou insaturé, non substitué ou substitué, contenant facultativement un autre hétéroatome choisi parmi l'oxygène ou le soufre ou
l'azote, ou R⁸ est hydrogène et R⁹ est amidino ; ou NR⁸R⁹ représente propargylamino,
R¹⁰, R¹¹ sont indépendamment hydrogène, alkyle en C1-C6, ou R¹⁰ et R¹¹, conjointement avec l'atome d'azote forment un cycle penta- ou hexa-monohétérocyclique saturé ou insaturé ; pour la prévention ou le traitement d'une maladie due à une fibrose d'organe choisie dans le groupe constitué de : une fibrose rénale ; une fibrose cardiaque ; une fibrose pancréatique ; une fibrose pulmonaire ; une fibrose vasculaire ; une fibrose de la moelle osseuse, une fibrose hépatique ; ou une fibrose d'organe due à une sclérose systémique.

2. Composé pour utilisation selon la revendication 1, choisi dans le groupe constitué de :
17-bêta-(3-furyl)-5-bêta-androstane-3-bêta,14-bêta,17-alpha-triol ;
3-bêta-(2-hydroxyéthoxy)-17-bêta-(3-furyl)-5-bêta-androstane-14-bêta,17-alpha-diol ;
3-bêta-(2-aminoéthoxy)-17-bêta-(3-furyl)-5-bêta-androstane-14-bêta,17-alpha-diol ;
3-bêta-(3-aminopropoxy)-17-bêta-(3-furyl)-5-bêta-androstane-14-bêta,17-alpha-diol ;
3-bêta-(2-méthylaminoéthoxy)-17-bêta-(3-furyl)-5-bêta-androstane-14-bêta,17-alpha-diol ;
3-bêta-(2-(1-pyrrolidinyl)éthoxy)-17-bêta-(3-furyl)-5-bêta-androstane-14-bêta,17-alpha-diol ;
3-bêta-(2-(3-(1-pyrrolidinyl)propoxy)éthoxy)-17-bêta-(3-furyl)-5-bêta-androstane-14-bêta,17-alpha-diol ;
3-bêta-(3-(1-pyrrolidinyl)propoxy)-17-bêta-(3-furyl)-5-bêta-androstane-14-bêta,17-alpha-diol ;
3-bêta-(2-(1-imidazolyl)éthoxy)-17-bêta-(3-furyl)-5-bêta-androstane-14-bêta,17-alpha-diol ;
3-bêta-(2-(2-imidazolin-2-yl)éthoxy)-17-bêta-(3-furyl)-5-bêta-androstane-14-bêta,17-alpha-diol ;
3-bêta-(2-(2-amidino)éthoxy)-17-bêta-(3-furyl)-5-bêta-androstane-14-bêta,17-alpha-diol ;
3-bêta-(2-(2-(1-pyrrolidinyl)éthoxy)éthoxy)-17-bêta-(3-furyl)-5-bêta-androstane-14-bêta,17-alpha-diol ;
3-bêta-(2-guanidinoéthoxy)-17-bêta-(3-furyl)5-bêta-androstane-14-bêta,17-alpha-diol ;
3-bêta-(3-guanidinopropoxy)-17-bêta-(3-furyl)-5-bêta-androstane-14-bêta,17-alpha-diol ;
3-bêta-(3-amino-2-hydroxypropoxy)-17-bêta-(3-furyl)-5-bêta-androstane-14-bêta,17-alpha-diol ;
3-bêta-(2,3-diaminopropoxy)-17-bêta-(3-furyl)5-bêta-androstane-14-bêta,17-alpha-diol ;
17-bêta-(3-furyl)-17-alpha-méthoxy-5-bêta-androstane-3-bêta,14-bêta-diol ;
17-bêta-(3-furyl)-17-alpha-(2-(1-pyrrolidinyl)éthoxy)-5-bêta-androstane-3-bêta,14-bêta-diol ;
17-bêta-(3-furyl)-17-alpha-(3-aminopropoxy)-5-bêta-androstane-3-bêta,14-bêta-diol ;
3-bêta-(2-(1-pyrrolidinyl)éthoxy)-17-bêta-(3-furyl)-17-alpha-méthoxy-5-bêta-androstan-14-bêta-ol ;
3-bêta,17-alpha-bis(2-(1-pyrrolidinyl)éthoxy)-17-bêta-(3-furyl)-5-bêta-androstan-14-bêta-ol ;
3-bêta,17-alpha-Bis(3-aminopropoxy)-17-bêta-(3-furyl)-5-bêta-androstan-14-bêta-ol ;
14-bêta,17-alpha-dihydroxy-17-bêta-(3-furyl)-5-bêta-androstan-3-one ;
3-guanidinoimino-17-bêta-(3-furyl)-5-bêta-androstane-14-bêta,17-alpha-diol ;
17-bêta-(4-pyridazinyl)-5-bêta-androstane-3-bêta,14-bêta,17-alpha-triol ;
3-bêta-(2-hydroxyéthoxy)-17-bêta-(4-pyridazinyl)5-bêta-androstane-14-bêta,17-alpha-diol ;
3-bêta-(3-aminopropoxy)-17-bêta-(4-pyridazinyl)-5-bêta-androstane-14-bêta,17-alpha-diol ;
3-bêta-(2-(1-pyrrolidinyl)éthoxy)-17-bêta-(4-pyridazinyl)-5-bêta-androstane-14-bêta,17-alpha-diol ;
3-bêta-(3-(1-pyrrolidinyl)propoxy)-17-bêta-(4-pyridazinyl)-5-bêta-androstane-14-bêta,17-alpha-diol ;
17-bêta-(4-pyridazinyl)-17-alpha-(3-aminopropoxy)-5-bêta-androstane-3-bêta,14-bêta-diol ;
3-bêta-(2-(1-pyrrolidinyl)éthoxy)-17-bêta-(4-pyridazinyl)-17-alpha-méthoxy-5-bêta-androstan-14-bêta-ol ;
3-bêta-(2-(1-pyrrolidinyl)éthoxy)-17-bêta-(4-pyridazinyl)-17-alpha-(3-amino-propoxy)-5-bêta-androstan-14-bêta-ol ;
14-bêta,17-alpha-dihydroxy-17-bêta-(4-pyridazinyl)-5-bêta-androstan-3-one ;
3-guanidinoimino-17-bêta-(4-pyridazinyl)-5-bêta-androstane-14-bêta,17-alpha-diol ;
14-bêta,15-bêta-époxy-17-bêta-(3-furyl)-5-bêta-androstane-3-bêta,17-alpha-diol ;
3-bêta-(2-hydroxyéthoxy)-14-bêta,15-bêta-époxy-17-bêta-(3-furyl)-5-bêta-androstan-17-alpha-ol ;
3-bêta-(3-aminopropoxy)-14-bêta,15-bêta-époxy-17-bêta-(3-furyl)-5-bêta-androstan-17-alpha-ol ;
3-bêta-(2-(1-pyrrolidinyl)éthoxy)-14-bêta,15-bêta-époxy-17-bêta-(3-furyl)-5-bêta-androstan-17-alpha-ol ;
3-bêta-(3-(1-pyrrolidinyl)propoxy)-14-bêta,15-bêta-époxy-17-bêta-(3-furyl)-5-bêta-androstan-17-alpha-ol ;
3-bêta-(2-(1-pyrrolidinyl)éthoxy)-17-bêta-(3-furyl)-17-alpha-méthoxy-14-bêta,15-bêta-époxy-5-bêta-androstane ;
17-alpha-hydroxy-17-bêta-(3-furyl)-14-bêta,15-bêta-époxy-5-bêta-androstan-3-one ;
3-guanidinoimino-17-bêta-(3-furyl)-14-bêta,15-bêta-époxy-5-bêta-androstan-17-alpha-ol ;
14-bêta,15-bêta-époxy-17-bêta-(4-pyridazinyl)-5-bêta-androstane-3-bêta,17-alpha-diol ;
et les dérivés 3-alpha des dérivés 3-bêta identifiés ci-dessus et également les thiodérivés 3-alpha et 3-bêta correspondants où Y = S.

3. Utilisation d'un composé tel que décrit dans la revendication 1 pour la fabrication d'un médicament pour la prévention et/ou le traitement d'une maladie due à une fibrose d'organe choisie dans le groupe constitué de : une fibrose rénale ; une fibrose cardiaque ; une fibrose pancréatique ; une fibrose pulmonaire ; une fibrose vasculaire ; une fibrose de la moelle osseuse, une fibrose hépatique ; ou une fibrose d'organe due à une sclérose systémique.

4. Utilisation selon la revendication 3, dans laquelle le composé préféré est le 17-bêta-(3-furyl)-5-bêta-androstane-3-bêta,14-bêta,17 alpha-triol.

5. Utilisation selon la revendication 3, dans laquelle la fibrose hépatique est due à une maladie virale, l'hépatite alcoolique, la stéatohépatite non alcoolique, la cirrhose ou le cancer du foie.

6. Utilisation selon la revendication 3, dans laquelle le composé est administré à une dose de 0,05 mg à 20 mg par jour.

7. Utilisation selon la revendication 3, dans laquelle le composé est administré à une dose de 0,5 mg à 15 mg.

8. Utilisation selon la revendication 3, dans laquelle le composé est administré à une dose de 5 mg à 10 mg.

9. Utilisation selon la revendication 3, dans laquelle le composé est administré dans un schéma de dose unique.

10. Utilisation selon la revendication 3, dans laquelle le composé est administré dans un schéma à dose multiple.

11. Utilisation selon la revendication 3, dans laquelle la composition est pour administration orale, intraveineuse, intramusculaire, intra-artérielle, intramédullaire, intrathécale, intraventriculaire, transdermique, transcutanée, sous-cutanée, intrapéritonéale, intranasale, entérale, topique, sublinguale ou rectale.

12. Utilisation selon la revendication 3, dans laquelle la composition est administrée localement sur le tissu malade après une opération chirurgicale.

13. Utilisation selon la revendication 3, dans laquelle la composition est revêtue sur l'endoprothèse ; incorporée dans une matrice à libération contrôlée ; ou incorporée dans des liposomes.
